(19) Europäisches Patentamt
European Patent Office
Office européen des brevets

(11) **EP 1 808 666 B1**

(12) **EUROPÄISCHE PATENTSCHRIFT**

(45) Veröffentlichungstag und Bekanntmachung des Hinweises auf die Patenterteilung:
**23.07.2014 Patentblatt 2014/30**

(51) Int Cl.:
*F41G 7/00* ⁽²⁰⁰⁶·⁰¹⁾

(21) Anmeldenummer: **06075083.3**

(22) Anmeldetag: **12.01.2006**

(54) **Testgerät zum Testen der Funktionsfähigkeit eines Warnsystems für sich nähernde Lenkwaffen**

Testing apparatus for checking the working ability of a warning system for approaching guided weapons

Appareillage de test pour vérifier la capacité de fonctionnement d'un système d'alerte pour armes guidées se rapprochant

(84) Benannte Vertragsstaaten:
**AT BE BG CH CY CZ DE DK EE ES FI FR GB GR HU IE IS IT LI LT LU LV MC NL PL PT RO SE SI SK TR**

(43) Veröffentlichungstag der Anmeldung:
**18.07.2007 Patentblatt 2007/29**

(73) Patentinhaber: **RUAG Schweiz AG**
**6032 Emmen (CH)**

(72) Erfinder:
• **Betschart, Christoph**
**3800 Interlaken (CH)**
• **Wepfer, Benjamin**
**3270 Aarberg (CH)**

(74) Vertreter: **Fischer, Franz et al**
**IPTO AG**
**International Patent and Trademark Office**
**Schwarztorstrasse 31**
**Postfach 5135**
**3001 Bern (CH)**

(56) Entgegenhaltungen:
**US-A- 5 416 332        US-A- 5 636 992**
**US-A- 5 693 951        US-A- 5 850 285**
**US-A1- 2004 174 290    US-A1- 2005 029 394**
**US-A1- 2005 150 371**

**Beschreibung**

Die vorliegende Erfindung bezieht sich auf ein Testgerät zum Testen der Funktionsfähigkeit eines Warnsystems für sich nähernde Lenkwaffen gemäss des Anspruchs 1. Sie liegt damit auf dem Gebiet der

Selbstschutzsysteme, welche Flugzeuge, Fahrzeuge, Schiffe etc. und seine Insassen vor Angriffen schützen sollen. Diese Systeme umfassen Sensoren, die auf Emissionen reagieren, die von einem sich nähernden Waffensystem, wie einer Rakete oder Lenkwaffe, ausgehen. Hat ein solches System eine sich nähernde Lenkwaffe erkannt, kann ein Abwehrsystem aktiviert werden, das bewirkt, dass diese Rakete dem Ziel nicht gefährlich werden kann. Sicherheitshalber sollten diese Sensoren der Selbstschutzsysterne vor jedem Einsatz auf ihre Funktion getestet werden. Insbesondere bezieht sich die vorliegende Erfindung auf Testgeräte, die eine Gefahrensimulation simulieren und der Crew oder dem Wartungspersonal die Funktionsfähigkeit des Selbstschutzsystems bestätigen.

Die Öffentlichkeit wurde erstmals durch einen Raketenangriff am 29. November 2002 auf ein israelisches Zivilflugzeug in Kenia auf die Gefahren aufmerksam gemacht, die von IR-Raketen ausgehen. Nicht nur militärische, sondern auch zivile Flugzeuge sind dieser Gefährdung ausgesetzt, die von terroristischen Aktivitäten ausgehen. Die Gefahr hat durch die Verbreitung von Schulter abgefeuerten Raketen (MANPADS = "man-portable air defense"), "Stinger", SAM 6, SAM 18 in den letzten Jahren zugenommen, da sie auf dem Schwarzmarkt zu tausenden erhältlich sind. Diese Raketen besitzen ein wärmegeleitetes Zielsystem (IR), das die Rakete auf die Wärme der Triebwerke richtet. Auf Grund der Reichweite dieser Raketen, besteht die Gefahr im Wesentlichen in der Start- und Landephase.

Mit der Einführung von modernen Selbstschutz-Systemen auf Flugzeugen und Helikoptern kommen komplexe und empfindliche Umweltsensoren zum Einsatz, die für die Detektion von schnell nahenden Gefahren nötig sind. Die Patentanmeldung US 2005/0150371 A1 beschreibt ein Gegenmassnahmen-System für die Verteidigung von Flugzeugen gegen Raketenangriffe. Das System umfasst einen Detektor, der sich am Boden befindet, alternativ jedoch auch am Flugzeug montiert sein kann. Weist das System eine sich nähernde Rakete nach, wird vom Flugzeug-Subsystem eine Wolke von fluoreszierenden Nanokristallen ausgestreut. Die entstehende Wolke von Nanokristallen wird danach durch Bestrahlung, z.B. durch Laser, aktiviert um für die Lenkwaffe einen Anlock-Hot Spot zu erzeugen und sie vom Flugzeug abzulenken. Die Patentanmeldung US 2005/0029394 A1 beschreibt ein eingelassenes Luftverteidigungssystem (conformal air defense system) das an der Aussenseite eines Flugzeuges montiert werden kann. Das System enthält einen Sensor und auch ein Gegenmassnahmensystem. Weitere derartige System sind z.B. beschrieben in US 2004/0174290 A1 und US Patent 5 850 285. Testsvorrichtungen für die Sensorsysteme werden in diesen Dokumenten nicht erwähnt.

Jedes Triebwerk einer fliegenden Lenkwaffe emittiert neben IR- auch UV-Strahlen. Ein "Missile Approach Warning Sensor" (MAWS) sucht daher die Umgebung permanent nach IR und/oder UV verdächtigen Quellen ab. Entdeckt der MAWS eine verdächtige Quelle, analysiert ein spezieller Rechenalgorithmus den Intensitätsverlauf der Quelle. Eine sich nähernde Missile verrät sich durch ein spezifisches "Missile-Profil", welches an Intensität zunimmt. Ein MAWS muss diese Bedrohung schnell erfassen, damit nötige Gegenmassnahmen rechtzeitig eingeleitet werden können. Die Richtung, aus welcher eine Gefahr herannaht, wird auf einem Display im Cockpit dargestellt.

Die regelmässige Funktionsüberprüfung von solchen Sensoren ist daher ein überlebenswichtiger Vorgang und wird in der Regel vor jeder Mission durchgeführt.

Die bisherige Stimulus-Technologie, basierend auf heissen Quellen (mit IR, VIS und UV Anteilen), kann aufgrund hohem Energiebedarf und Überhitzungsgefahr die Anforderungen für ein Handgerät nur beschränkt erfüllen. Die gemäss dem Stand der Technik verwendeten Halogen- und Gasentladungslampen sind für Handgeräte nicht gut geeignet. Diese Nachteile könnten mit einer zweckmässigen UV-Quelle umgangen werden, die auch für ein Hand-Held MAWS-Testgerät geeignet wäre.

US-5,693,951 beschreibt ein Gerät, mit dem eine annahende Lenkwaffe simuliert werden kann. Das Gerät verwendet eine Halogenlampe, die Licht ausgibt, das je nach fliessendem Strom andere Anteile von UV und IR aufweist. Damit kann die Signatur einer Lenkwaffe von Start (hohe UV-Intensität) bis Brennende (wenig UV; hoher, abnehmender IR Anteil) nachgebildet werden. Das Gerät wird am Boden aufgestellt und erzeugt bei Annäherung ein Lichtsignal, das von einem MAWS in einem heranfliegenden Luftfahrzeug detektiert werden kann. Wegen der relativ grossen Distanz zwischen Gerät und Luftfahrzeug wie auch wegen des verwendeten Leuchtmittels werden grosse Energiemengen benötigt, die allenfalls einen Transport auf einem Fahrzeug gestatten und entweder Stromversorgung aus dem Netz oder leistungsfähige und damit voluminöse und schwere Batterien erfordern.

Es besteht deshalb ein Bedürfnis nach einem Testgerät, das die UV-Strahlen einer Missile simulieren kann, das leicht, kompakt, mobil und einfach zu bedienen ist, aber auch unter härtesten Umweltbedingungen zuverlässig arbeitet. Ein derartiges Gerät soll diese Funktion als autonomes Hand-Held Gerät erfüllen.

Es ist daher Aufgabe der vorliegenden Erfindung ein solches Gerät zur Verfügung zu stellen.

Diese Aufgabe wird durch das Testgerät, auch genannt Missile Approach Warning Tester, abgekürzt MAWST, gemäss Anspruch 1 gelöst. Die weiteren Ansprüche geben bevorzugte Ausführungsformen an.

Die Erfindung wird weiter anhand eines bevorzugten Ausführungsbeispiels unter Bezugnahme auf Figuren erläutert.

Kurze Beschreibung der Zeichnungen

Fig. 1 zeigt den Unterschied eines konventionellen Testgerätes mit einer Lampe zum erfindungsgemässen Gerät.

Fig. 2 zeigt die Intensitätskurve der Emission einer sich nähernden Lenkwaffe.

Fig. 3 stellt eine Messanordnung mit einem erfindungsgemässen MAWST dar.

Fig. 4 zeigt ein Blockschaltbild, das die wesentlichen Elemente eines erfindungsgemässen Testgeräts enthält.

Fig. 5 zeigt einen einsetzbaren Regelkreis eines Microcontroller.

Fig. 6 zeigt ein Diagramm des Intensitätsverlaufs der verwendeten UVC-LED

Fig. 7 zeigt das in ein Gehäuse eingebautes MAWST.

Fig. 8 zeigt eine weitere Ausgestaltung des erfindungsgemässen MAWST mit einer geregelten Lichtleistung.

Fig. 9 zeigt eine Ausführungsform mit einer Ausgangsleistungskompensation mittels einer automatischen Distanzvermessung.

Fig. 10 zeigt eine weitere Ausführungsform eines MAWST, bei welchem das optische Signal eines Missile Approach Simulator sehr nahe am Missile Approach Warning Sensor eingespeist wird.

Die Fig. 11 bis 13 stellen Struktogramme dar, welche als Grundlage zur Programmierung des Microcontrollers dienten.

Die effizienteste Leuchtquelle ist nach heutigem Stand der Technik die Light Emitting Diode (LED), die in vielen Produkten als Beleuchtungskörper oder Kontrolllampen zum Einsatz gelangen. Hohe Lichtausbeute, lange Lebensdauer und hohe Effizienz zeichnen diese Technologie aus und hat sich seit Jahrzehnten bewährt. Die Hersteller liefern viele verschiedene Grössen, Leistungsklassen und Wellenlängen.

Es wurde gefunden, dass die LED-Technologie für ein MAWS-Testgerät (MAWST) geeignet ist und derartige LEDs bereits entwickelt wurden.

Während leistungsstarke IR-LED im Handel erhältlich sind, gibt es jedoch bei den Wellenlängen im UV-Bereich nur wenige Produkte mit kürzerer Wellenlänge, die für MAWSTs geeignet sind. Sie haben generell folgende Nachteile: Tiefer Wirkungsgrad, Keine hohen Ausgangsleistungen, Lebensdauer im Vergleich zu Standard-LED relativ kurz, es gibt nur wenige Bezugsquellen für LEDs, die unter 300 nm emittieren. Vorgeschlagene Anwendungen für diese neuen UVC-LEDs liegen im Entkeimen von Flüssigkeiten (Trinkwasseraufbereitung oder Schwimmbeckenreinigung). Es werden aber auch militärischen Anwendungen geprüft, z.B. für die Datenübertragung. Man sendet die Daten mittels UV-Strahlung in den Himmel. Die Strahlen werden dann diffus reflektiert. Diese Übertragungsart dürfte nur für kurze Distanzen (innerhalb Gefechtsstand) geeignet sein. Ihre Anwendung in Missile Approach Simulatoren wurde bisher nicht vorgeschlagen.

Für die vorliegende Erfindung wurde eine handelsübliche UVC-LED verwendet, welche Lichtwellen im Bereich um ca. 265nm Wellenlänge emittiert. Da diese Wellenlänge in der Bandbreite für UV-MAW-Sensoren (die primär im Solar-Blind Bereich detektieren) auch noch enthalten sind, sind solche UVC-LED für diese Anwendung ideal. Die relativ kurze Lebensdauer der gegenwärtig erhältlichen UVD-LEDs ist für die die Simulation kein gravierender Nachteil, da eine verwendete UVC-LED für einen Test nur während einigen Sekunden in Betrieb genommen wird.

Damit das MAWS-Testgerät (MAWST) bezüglich des UV einer realen Lenkwaffe mit ihren Eigenschaften möglichst nahe kommt, wurden Messungen an einem integriertem Selbstschutzsystem in eine Flugzeug durchgeführt und verifiziert.

Das MAWST wird so programmiert, dass bei Aktivierung ein UV-Emissionsprofil einer sich nähernden Lenkwaffe mittels einer UV-Quelle, erfindungsgemäss mit einer UVC-LED, simuliert wird. Bei diesem Profil ist der Intensitätsverlauf massgebend, damit der Sensor die Lichtquelle auch wirklich als Gefahr wahrnehmen kann. Die vom Sensor erkannte Gefahr und ihre Richtung werden vom MAWS ins Cockpit übermittelt, wo der Piloten auf einem Display auf die Gefahr aufmerksam gemacht wird, wobei im Gefahrenfall sofort Gegenmassnahmen getroffen werden (für Lenkwaffen meist Flares, Leuchtkugeln welche als störende Wärmequell wirken sollen). Da der interne Selbsttest aber vor einem Abflug nicht alle Teile des MAWS auf seine Funktion überprüfen kann, braucht es für den Sensor einen externen Test. Dieser soll die Funktionalität des Sensor noch einmal beweisen bzw. Fehlerquellen auf nicht elektronisch zu überprüfenden Teilen wie verschmutztes Schutzglas vor einem Sensor zu vermindern.

Das MAWST muss also, eine Lenkwaffe bezüglich der UV-Abstahlung so simulieren, dass Gefahr vom Sensor auch wirklich als solche erkannt wird. Da die meisten MAWS im UV-Bereich arbeiten, benötigt man für ein Testgerät eine Lichtquelle in diesem Bereich. Um die auf dem Markt erhältlichen MAWS zu stimulieren, benötigt man eine Quelle im UVC Bereich (genauere Erläuterungen siehe weiter unten in der vorliegenden Beschreibung).

Die Stromversorgung des MAWST, das als autonomes Gerät funktionieren soll, ist vorzugsweise ein Akkumulator oder eine nichtwiederaufladbare Batterie.

Als Bedienelement kann eine Taste vorgesehen werden, mit welcher durch schliessen eines Stromkreises während der nötigen Zeitdauer das Profil gestartet bzw. beendet werden kann.

Wie bei andern ähnlichen Geräten wird beim MAWST ein programmierbarer Controller eingesetzt um Unlinearitäten oder andere Einflüsse zu kompensieren.

Die Sollwerte eines UV-Emissionsprofils einer sich annähernden generischen Lenkwaffe stammen aus gerechneten Profilen oder aus echten Aufzeichnungen, die in den MAWST geladen werden können. Die Werte beschreiben einen Intensitätsverlauf einer sich annähernde Lenkwaffe. Die Kurvenformen beruhen grob auf einer quadratischen Funktion, da sich die Lichtleistung auch quadratisch zur Distanz verhält. Die Regelstrecke (ausserhalb und/oder innerhalb des Mikrocontrollers) besteht aus einem Verstärkerglied und der UV-LED. Der Verstärker ist notwendig, da sich der Arbeitspunkt der UV-LED erst oberhalb der Speisespannung des Mikrocontrollers befindet. Spannung, Strom und Temperatur der UV-LED werden als Istwerte an den Controller zurückgeführt und könne für Regelzwecke verwendet werden.

Die oben beschriebene Version des MAWST ist eine kostengünstige Version. E können noch zusätzliche Funktionen in das Gerät integriert werden, die bei einer Serieproduktion auch berücksichtigt werden können.

Die Kalibration des MAWST erfolgt durch eine externen Einrichtung.

UV-LED

Als UVC-LED wurden UVTOP® LEDs von Sensor Electronic Technology, Inc. 1195 Atlas Road Columbia, SC 29209, USA verwendet. Für das Produkt UVTOP-265 wird eine Spitzenwellenlänge mit 263 +/-7nm angegeben. Dies wurde durch eine Messung der Anmelderin bestätigt. Auch die Spectrum Half Width von 12-20nm wurde eingehalten. Andere derartige Produkte, welche die Voraussetzungen erfüllen, können jedoch auch verwendet werden.

Für die Ausgangsleistungs-Messungen wurde ein Universal Lichtleistungs-Messgerät (Model 841-PE, UC-Sensor 818) von Newport (Newport Corporation, 8 East Forge Parkway Franklin, MA 02038 USA) verwendet, an welchem kalibrierte Power-Sensoren angehängt werden können. Das Datenblatt des Sensors und der Anzeigeeinheit ist auf der Website www.newport.com ersichtlich.

Die Entfernung des MAWST vom MAW-Sensor am ISSYS ist im weiteren von Bedeutung, da die Lichtleistung bei zunehmender Distanz quadratisch abnimmt.

In einer Ausführungsform des Testgeräts ist deshalb ein Distanzmesser eingebaut, der bei der Anwendung des Testgeräts in eingeschaltetem Zustand die Distanz zwischen der UVC-LED und dem Sensor des MAWS vermisst. Abhängig von der Distanz wird die optische Ausgangsleitung durch den programmierbaren Controller so gesteuert, dass die Leistung der UVC-LED innerhalb der gewünschten Spezifikation liegt. Der Anwender muss sich nicht mehr um die Distanz zum Sensor kümmern.

In einer speziellen Ausführungsform des Testgeräts wird vor der UVC-LED ein Mess-Sensor angeordnet ist, der die Intensität der emittierten Strahlung misst und durch den Controller regelt, wobei die Anordnung mit dem Mess-Sensor einen halbtransparenten Spiegel enthält, der einen Teil des optischen Signals für die Messung zum Mess-Sensor abzweigt.

Eine weitere Ausführungsform des Testgeräts weist zusätzlich ein Lichtleiterkabel auf, das vor der UVC-LED zur Weiterleitung des abgegebene optischen Signals angeordnet ist. Das optische Signal kann dadurch zwecks einer abgeschirmten Simulation, nahe am Sensor des MAWS eingespeist werden.

Hardware

Das Herzstück des MAWST ist ein programmierbarer Controller. Er steuert und überwacht die Baugruppen auf der Leiterplatte.

Ansteuerung der UV-Quelle

Wie aus den obigen Ausführungen ersichtlich ist, wird die Lichtleistung über den Strom gesteuert. Die Schaltung beruht auf dem Prinzip einer geregelten Stromquelle mit einem Operationsverstärker. Der Strom wird über einen Shunt auf den invertierten Eingang am OPAMP zurückgeführt. Der Sollwert wird auf den positiven Eingang geführt. Die Ausgangsspannung fährt nun also so lange hoch, bis die Differenz zwischen positivem und invertiertem Eingang Null ergibt.

Gehäuse

Eine Variante eines Gehäuses besteht aus glasfaserverstärktem Kunststoff (GFK), das eine einfache Lösung für den Wechsel der Batterie aufweist.

Software

Die Ansteuerung der eingesetzten Baugruppen und vor allem der UV-LED erfolgt über den programmierbaren Controller. Dies bedingt eine geeignete Firmware im Controller und Profildaten für die korrekte Ansteuerung und Überwachung.

Das Ausführungsbeispiel wird anhand der beiliegenden Zeichnungen näher erläutert.

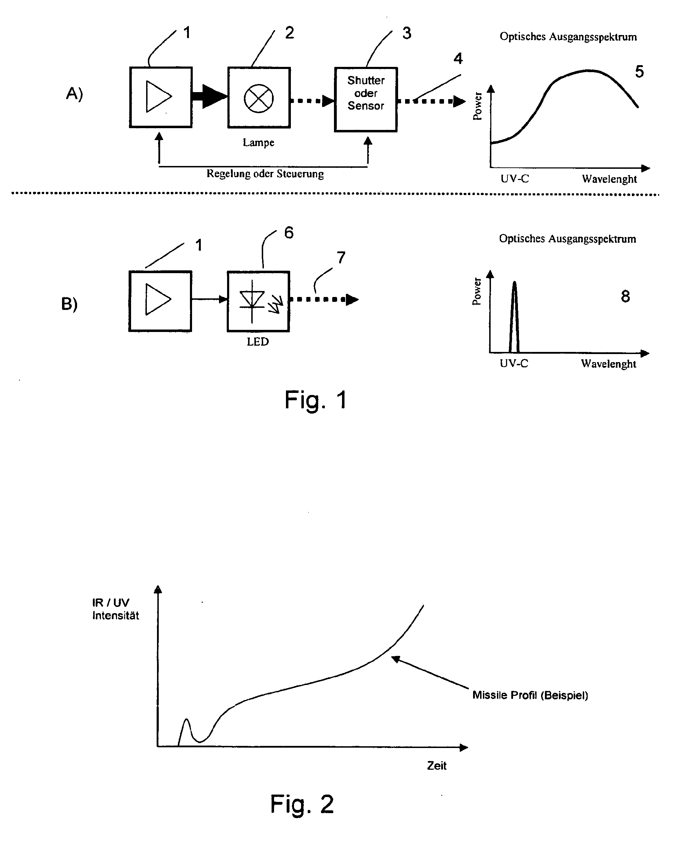

Fig. 1 zeigt den Unterschied eines konventionellen Testgerätes mit einer Lampe zum erfindungsgemässen LED Gerät. Da die meisten Missile Sensoren im Infrarot- und Ultraviolett-Bereich arbeiten, kamen bisher diverse Leuchtkörperlösungen mit Halogen- und diverse Gasentladungs-Lampen für Testgeräte zum Einsatz. Ein beispielhafte Anordnung ist unter A) in Fig. 1 dargestellt. 1 stellt die Elektronik, einschliesslich Speisung und Controller dar, der die Lampe 2 steuert. Es waren aufwändige optische Filter, Shutter 3 nötig, deren Ausgangsleistung im verlangten Spektralbereich geregelt werden müssen um die gewünschte Strahlungsintensität 4 zu erreichen. Auch die Unlinearitäten sowie schnelle Intensitätsveränderungen mit Steuervorrichtungen mussten von der Elektronik berücksichtigt werden. Diese Lösungen sind teuer, benötigen viel Energie, sind in der Anwendung unpraktisch und oft nicht zuverlässig. Unter B) in Fig. 1 wir die erfindungsgemässe Anordnung dargestellt. Sie umfasst ebenfalls eine Elektronik 1, die allein die LED 6 zu regeln hat, damit die gewünschte Strahlungsintensität 7 erreicht wird. Weiter ist aus der Darstellung schematisch der Unterschied der Ausgangsspektren 5 bzw. 8 ersichtlich.

Fig. 2 zeigt die Intensitätskurve einer sich dem MAWS nähernden Lenkwaffe. Die Intensität gemäss dieser Kurve muss durch die Elektronik des erfindungsgemässen MAWST simuliert werden, wobei ein berechnete Kurve verwendet wird.

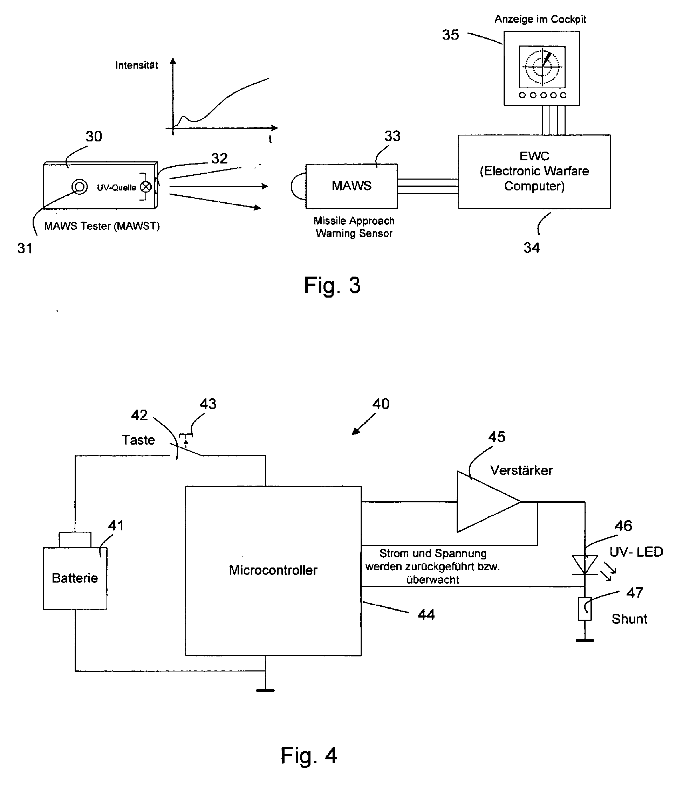

Fig. 3 stellt eine Messanordnung mit einem erfindungsgemässen MAWST 30 dar. Durch den Druck des Bedieners auf die Taste 31 wird die UVC-LED 32 aktiviert und strahlt mit einem vorgegeben Intensitätsmuster auf den Sensor 33 des MAWS. Das Signal wird durch die Elektronik 34 (hier EWC) verarbeitet und im Testfall lediglich an eine Anzeige 35 im Cockpit weitergeleitet, wo die Funktionsfähigkeit des MAWS bestätigt wird.

Fig. 4 zeigt ein Blockschaltbild, das die wesentlichen Elemente eines erfindungsgemässen Testgeräts 40 enthält. Die Stromquelle 41 sorgt dafür, das das MAWST als autonomes Gerät funktionieren kann. Die Stromquelle 41 besteht aus einem Akkumulator oder einer Batterie und liefert dem Microcontroller 44 die nötige Energie für die UV-LED 46 und für die Steuerung, wenn die Einschaltvorrichtung 42 vom Anwender mit der Taste 43 getätigt wird. Die Lichtleistung wird über den Strom gesteuert, wobei ein Operationsverstärker 45 (OPAMP) eingesetzt wird, der als Regler benutzt werden kann. Der Strom wird über einen Shunt 47 auf den invertierten Eingang am OPAMP zurückgeführt. Der Sollwert wird auf den positiven Eingang geführt.

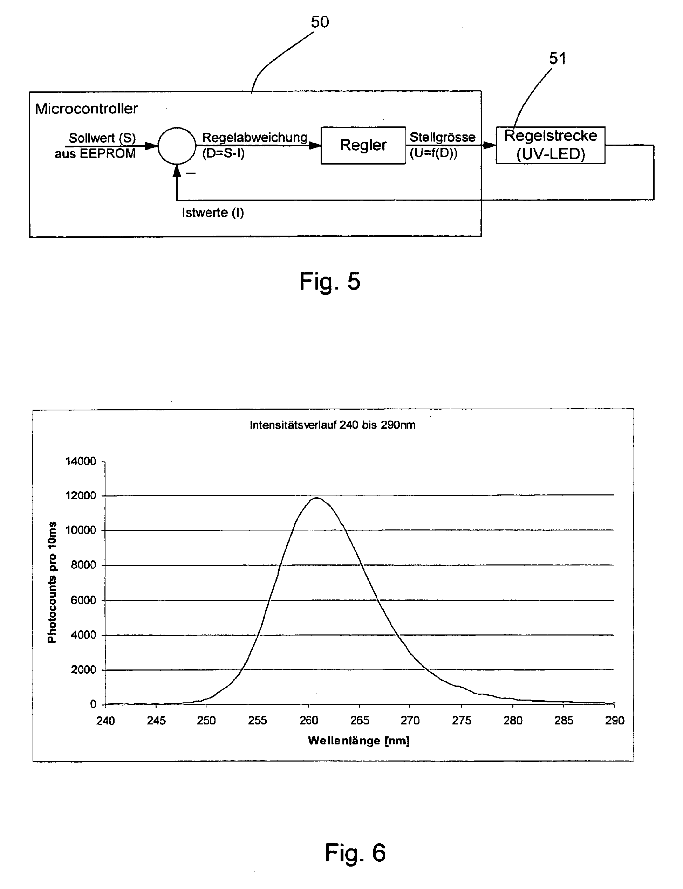

Fig. 5 zeigt einen einsetzbaren Regelkreis eines Microcontroller 50. Die Sollwerte (S) setzen sich aus einem generischen Profil zusammen. Für das Funktionsmuster wird in der Regel nicht ein Profil einer bestehenden Lenkwaffe eingesetzt, sondern ein gerechnetes Profil. Die Werte beschreiben einen Intensitätsverlauf einer sich annähernden Lenkwaffe. Die Kurvenform beruht grob auf einer quadratischen Funktion, da sich die Lichtleistung auch quadratisch zur Distanz verhält. Die Regelstrecke 51 (ausserhalb des Mikrocontrollers) besteht aus einem Verstärkerglied und der UV-LED. Der Verstärker ist notwendig, da die UV-LED erst oberhalb der Speisespannung des Mikrocontrollers anfängt zu leiten. Die Ansteuerung über einen "normalen" Output des Controllers ist also nicht möglich. Spannung, Strom und Temperatur der UV-LED werden als Istwerte an den Controller zurückgeführt.

Fig. 6 zeigt ein Diagramm des Intensitätsverlaufs der verwendeten UVC-LED des Typs UVTOP-265 von Sensor Electronic Technology, Inc., Columbia, SC 29209, USA.

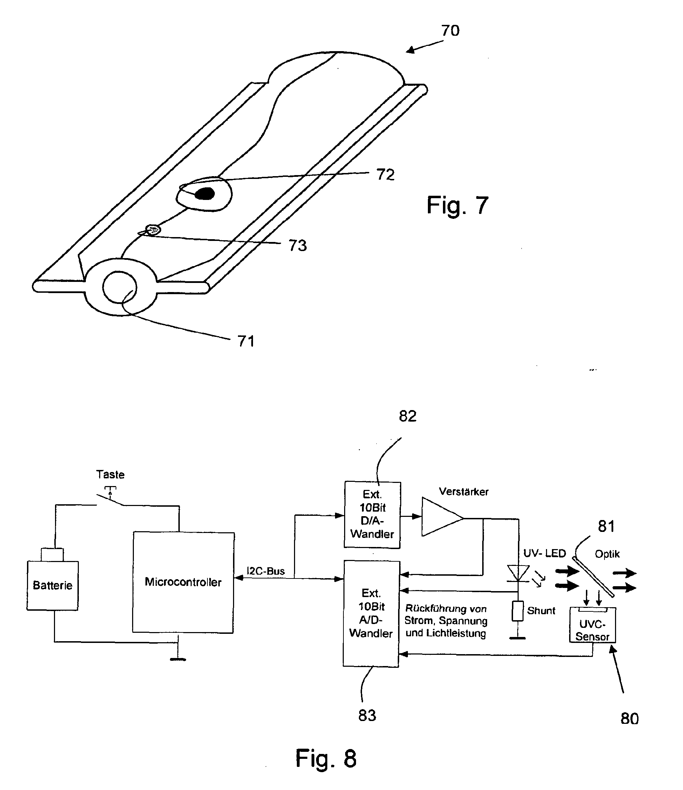

Fig. 7 zeigt ein in ein Gehäuse 70 eingebautes MAWST mit der UVC-LED 71, der Taste 72 und einer Kontrollleuchte 73.

Fig. 8 zeigt eine weitere Ausgestaltung des erfindungsgemässen MAWST mit einer geregelten Lichtleistung. Die Anordnung entspricht derjenigen von Fig. 4, wobei jedoch zusätzlich die Intensität des UV-Strahles durch eine Mess-Sensoranordnung 80 gemessen und durch zusätzliche elektronische Bauteile 82, 83 geregelt wird. Durch einen durchlässigen Spiegel 81 als Optik wird ein kleiner Teil des UV-Lichtes, das für dem MAWS-Sensor bestimmt ist, auf den Mess-Sensor abgelenkt, der entsprechende Signale zum Bauteil 83 sendet. Dadurch wird den Veränderungen an der UVC-LED Rechnung getragen, die durch äussere Einflüsse oder Alterung auftreten können. Diese Ausführungsform ist nicht nur auf die Verwendung einer UVC-LED beschränkt, sondern kann auch bei MAWSTs angewendet werden, die im IR Bereich arbeiten und/oder Lampen oder andere Strahlungsquellen verwenden.

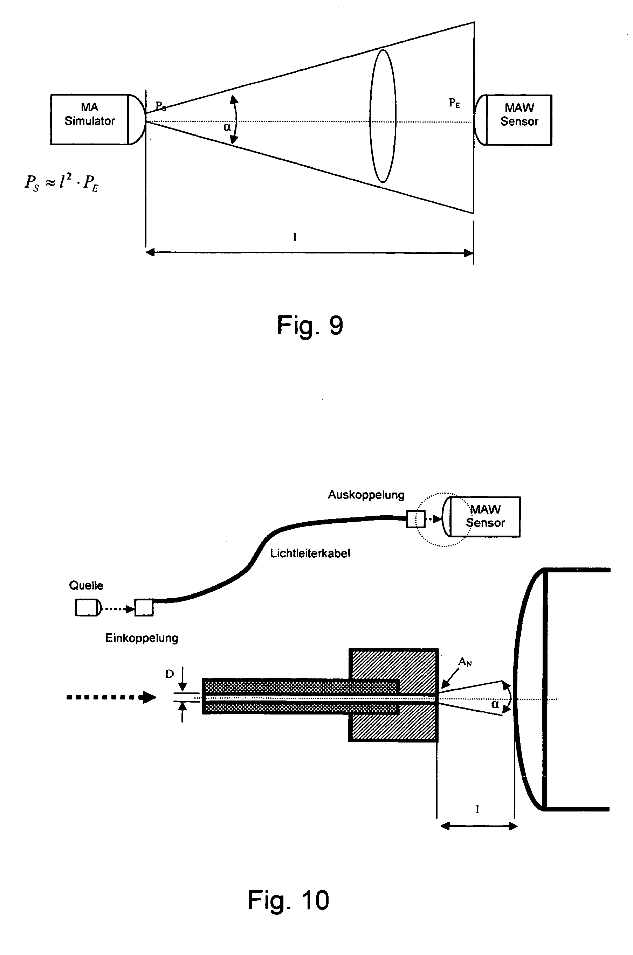

Fig. 9 zeigt eine Ausführungsform mit einer Ausgangsleistungskompensation mittels einer automatischen Distanzvermessung. Das MAWST detektiert anhand der optischen Eingangsleistung $P_E = f(t)$ eine Bedrohung. Diese optische Leistung ist ein wichtiger Parameter und muss daher innerhalb der Spezifikation liegen. Die eintreffende Leistung am Sensor bei einer Missile Approach Simulation steht im Verhältnis : $P_E \approx P_S / I^2$. Die Distanz hat einen grossen Einfluss auf die Eingangsleistung und ist somit eine kritische Grösse. Im Missile Approach Simulator ist ein Distanzmesser eingebaut, der die Strecke zwischen Simulator und MAW Sensor ausmisst. Abhängig von der Distanz wird die optische Ausgangsleistung am Simulator angepasst. Dadurch wird sichergestellt, dass die optische Leistung am MAW Sensor immer innerhalb der gewünschten Spezifikation liegt. Der Anwender muss sich nicht mehr um die Distanz zum Sensor kümmern. Diese Ausführungsform ist nicht nur auf die Verwendung einer UVC-LED beschränkt, sondern kann auch bei MAWSTs angewendet werden, die im IR Bereich arbeiten und/oder Lampen oder andere Strahlungsquellen verwenden. In der Darstellung bedeutet: $P_S$ = Sendeleistung; $P_E$ = Empfangsleistung; I = Distanz Sender - Empfänger

Fig. 10 zeigt eine weitere Ausführungsform eines MAWST, bei welchem das optische Signal eines Missile Approach Simulator sehr nahe am Missile Approach Warning Sensor eingespeist wird. Diese minimale Distanzvorgabe ermöglicht die Durchführung von abgeschirmten Simulationen. Da die Optik eines MAW Sensor fix auf eine grosse Distanz fokussiert ist, muss bei der Einspeisung des optischen Signals aus einer kurzen Distanz die Optik überlistet werden. Hierzu wird der Einsatz von Lichtleiterkabel mit einer optischen Transmission von 160 - 10'000 nm vorgeschlagen. Bedingt durch die Numerische Apertur und den Faserdurchmesser kann die optische Leistung angepasst und der Austrittswinkel berechnet werden. Der MAW Sensor "sieht" das optische Signal bei einem kleinen Faserdurchmesser trotz kleiner Distanz scharf. Der untere Teil der Zeichnung zeigt eine Detaildarstellung des Teils, der sich im punktierten Kreis der oberen Übersichtszeichnung befindet.

Diese Ausführungsform ist nicht auf die Verwendung einer UVC-LED beschränkt, sondern kann auch bei MAWSTs angewendet werden, die im IR Bereich arbeiten und/oder Lampen oder andere Strahlungsquellen verwenden. In dieser Darstellung ist:

$$\alpha = 2 \cdot \arcsin\left(\frac{A_N}{\eta}\right)$$

AN: Numerische Apertur der Faser; $\eta$ : Brechzahl (Luft = 1,00, Quarz = 1,46); D: Durchmesser Glasfaser resp. Faserbündel; $\alpha$: Optischer Austrittswinkel; I: Distanz Auskoppelung - MAW Sensor

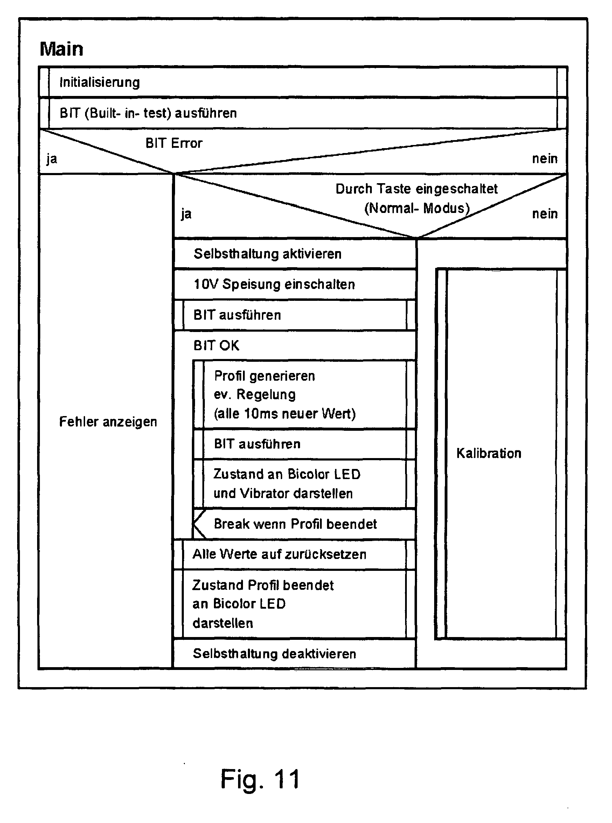

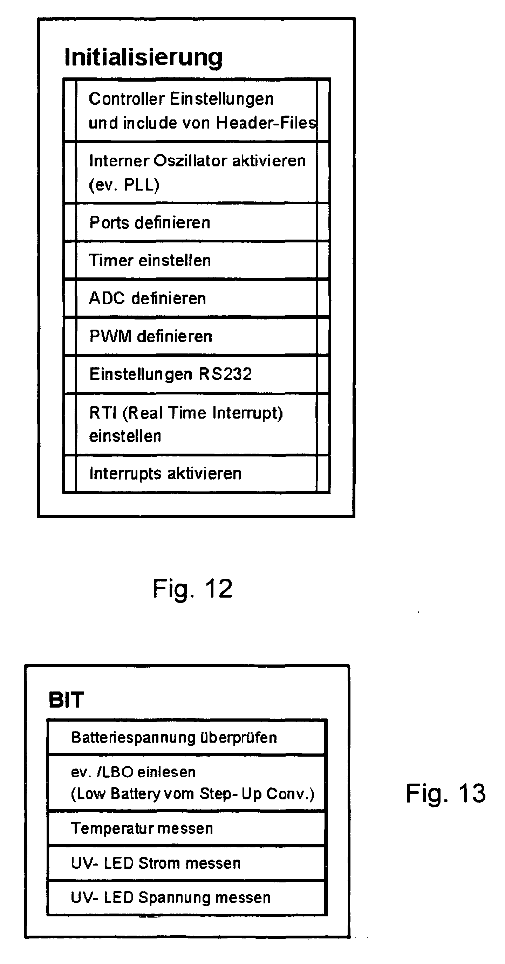

Die Fig. 11 bis 13 stellen Struktogramme dar, welche als Grundlage zur Programmierung des Controllers dienten, damit die Ansteuerung der eingesetzten Baugruppen und vor allem der UV-LED reibungslos funktioniert.

## Patentansprüche

1. Testgerät (30, 40) zum Testen der Funktionsfähigkeit eines Warnsystems für sich nähernde Lenkwaffen im Solar-Blind Bereich UV-C, insbesondere für Flugzeuge, Fahrzeuge und Schiffe, das einen UV-Sensor enthält, wobei der UV-sensor auf die Strahlenemission einer sich nähernde Lenkwaffe reagiert und Schutzmassnahmen auslöst, indem der UV-Sensor durch eine Strahlenemission des Testgeräts stimuliert wird, **dadurch gekennzeichnet, dass** das Testgerät folgende Komponenten enthält:

   eine UVC-LED (32, 46), die UV-Licht im UV-C-Bereich von 240 bis 290 nm aussendet, und einen programmierbaren Controller (44), der zumindest die UVC-LED ansteuert.

2. Testgerät nach Anspruch 1, **dadurch gekennzeichnet, dass** es weiter ein Einschaltmittel und eine Stromquelle enthält, wobei die Stromquelle bevorzugt ein Akkumulator oder eine Batterie ist.

3. Testgerät nach Anspruch 1 oder 2, **dadurch gekennzeichnet, dass** die UVC-LED im Bereich von 250 bis 270 nm und mit einem Intensitätsmaximum von etwa 261 nm arbeitet.

4. Testgerät nach einem der Ansprüche 1 bis 3, **dadurch gekennzeichnet, dass** es ein Anzeigemittel aufweist, welches durch den Controller angesteuert wird und anzeigt, wenn die UVC-LED eingeschaltet ist.

5. Testgerät nach Anspruch 4, **dadurch gekennzeichnet, dass** das Anzeigemittel eine Kontrollleuchte, ein Vibramotor oder ein akustisches Mittel ist.

6. Testgerät nach einem der Ansprüche 1 bis 5, **dadurch gekennzeichnet, dass** der Controller so programmiert ist, dass er ein berechnetes oder frei programmiertes Profil erzeugt, das dem Intensitätsverlauf einer sich nähernden Lenkwaffe entspricht.

7. Testgerät nach einem der Ansprüche 2 bis 5, dadurch gekennzeichnet, dass das Einschaltmittel durch eine Taste bedient wird, wobei das Einschaltmittel durch den Controller so angesteuert wird, dass auf Tastendruck eine vorbestimmte Einschaltdauer mit dem programmierten Intensitätsprotokoll erzielt wird.

8. Testgerät nach einem der Ansprüche 1 bis 7, **dadurch gekennzeichnet, dass** im Testgerät ein Distanzmesser eingebaut ist, der bei der Anwendung des Testgeräts in eingeschaltetem Zustand die Distanz zwischen der UVC-LED und dem Warnsystem misst, wobei die UVC-LED durch den Controller so gesteuert wird, dass die Leistung der UVC-LED in Abhängigkeit der Distanz entsprechend den Vorgaben für das zu testende System optimal eingestellt ist.

9. Testgerät nach einem der Ansprüche 1 bis 8, **dadurch gekennzeichnet, dass** vor der UVC-LED ein Mess-Sensor angeordnet ist, der die Intensität der emittierten Strahlung misst und durch den Controller regelt, wobei die Anordnung mit dem Mess-Sensor einen halbtransparenten Spiegel enthält, der einen Teil des optischen Signals für die Messung zum Mess-Sensor abzweigt.

10. Testgerät nach einem der Ansprüche 1 bis 9, **dadurch gekennzeichnet, dass** es zusätzlich ein Lichtleiterkabel aufweist, das vor der UVC-LED zur Weiterleitung des abgegebene optischen Signals angeordnet ist, damit das optische Signal vorzugsweise zwecks einer abgeschirmten Simulation, nahe am Sensor des Warnsystems eingespeist werden kann.

## Claims

1. Test apparatus (30, 40) for testing the operability of a warning system for approaching guided missiles in the solar-blind range, UV-C, especially for aircraft, vehicles, and ships comprising an UV sensor, the UV sensor is reacting to the radiation emission of an approaching guided missile and is triggering protective measures in that the UV sensor is stimulated by a radiation emission of the test apparatus, **characterized in that** the test apparatus comprises the following components:

   a UVC-LED (32, 46) which emits UV light in the range from 240 to 290 nm, and
   a programmable controller (44) which activates at least the UVC-LED.

2. Test apparatus according to claim 1, **characterized in that** it comprises a switching-on means and a current source, the current source preferably being a secondary cell or a battery.

3. Test apparatus according to claim 1 or 2, **characterized in that** the UVC-LED operates in the range from 250 to 270 nm and with a maximum intensity of about 261 nm.

4. Test apparatus according to one of the claims 1 to 3, **characterized in that** it has an indicator means which is activated by the controller and indicates when the UVC-LED is switched on.

5. Test apparatus according to claim 4, **characterized in that** the indicator means is a pilot light, a vibramotor, or an acoustic means.

6. Test apparatus according to one of the claims 1 to 5, **characterized in that** the controller is programmed in such a way that it generates a computed or freely programmed profile corresponding to the intensity course of an approaching guided missile.

7. Test apparatus according to one of the claims 2 to 5, **characterized in that** the switching-on means is operated by a button, the switching-on means being so triggered by the controller that upon pressing the button, a predetermined switched-on duration is achieved with the programmed intensity protocol.

8. Test apparatus according to one of the claims 1 to 7, **characterized in that** the test apparatus incorporates a range-finder which, when the test apparatus is used in switched-on condition, measures the distance between the UVC-LED and the warning system, the UVC-LED being so controlled by the controller that the output of the UVC-LED is optimally adjusted as a function of the distance corresponding to the default value for the system to be tested.

9. Test apparatus according to one of the claims 1 to 8, **characterized in that** a measuring sensor is disposed before the UVC-LED which measuring sensor measures the intensity of the emitted radiation and regulates it by means of the controller, the arrangement with the measuring sensor is containing a semi-transparent mirror which diverts part of the optical signal to the measuring sensor for the measurement.

10. Test apparatus according to one of the claims 1 to 9, **characterized in that** in addition a light-guide cable is disposed before the UVC-LED for relaying the emitted optical signal so that the optical signal, preferably for the purpose of a shielded simulation, can be fed in close to the sensor of the warning system.

## Revendications

1. Appareil testeur (30, 40) pour tester le fonctionnement d'un système d'alerte pour missiles guidés approchants, dans la région solaire aveugle, UV-C, en particulier pour les avions, les véhicules et les navires, comprenant un capteur d'UV, le capteur d'UV réagit à l'émission de rayonnement d'un missile approchant et déclenche des mesures de protection en ce que le capteur d'UV est stimulé par une émission de rayonnement de l'appareil testeur, **caractérisé en ce que** l'appareil testeur contient les composants suivants:

   une LED UV (32, 46), émettant de lumière UV dans la région de 240 à 290 nm, et
   un contrôleur programmable (44) qui entraîne au moins la LED UVC.

2. Appareil testeur selon la revendication 1, **caractérisé en ce qu'**il comprend en outre un switch d'enclenchement, et une source de courant, dans lequel la source de courant est de préférence un accumulateur ou une batterie.

3. Appareil testeur selon la revendication 1 ou 2, **caractérisé en ce que** la LED UV fonctionne dans l'étendue de 250 à 270 nm et avec un maximum d'intensité d'environ 261 nm.

4. Appareil testeur selon l'une des revendications 1 à 3, **caractérisé en ce qu'**il possède un moyen d'affichage qui est commandé par le contrôleur et indique le moment où la LED UV est allumée.

**5.** Appareil testeur selon la revendication 4, **caractérisé en ce que** le moyen d'affichage est un témoin lumineux, un vibramotor ou un moyen sonore.

**6.** Appareil testeur selon l'une des revendications 1 à 5, **caractérisé en ce que** le contrôleur est programmé pour générer un profil programmable librement ou calculée, lequel correspond au profil d'intensité d'un missile qui s'approche.

**7.** Appareil testeur selon l'une des revendications 2 à 5, **caractérisé en ce que** le switch d'enclenchement est actionné par une clé, ledit switch d'enclenchement est commandé par le contrôleur de sorte que, en appuyant sur la clé une duration prédéterminée avec le protocole de l'intensité programmée est atteinte.

**8.** Appareil testeur selon l'une des revendications 1 à 7, **caractérisé en ce que** dans l'appareil testeur un dispositif de mesure de distance est encastré, ce dispositif mesure, à l'état enclenché de l'appareil testeur, la distance entre l'UV-LED et le système d'alerte, dans lequel l'UV-LED est commandée par le contrôleur de sorte que la puissance de l'UV-LED, en fonction distance de référence pour le système testé, est réglé optimalement.

**9.** Appareil testeur selon l'une des revendications 1 à 8, **caractérisé en ce que** devant l'LED UV un capteur de mesure est disposé qui mesure l'intensité du rayonnement émis et la règle par le contrôleur, dans lequel le dispositif avec le capteur de mesure comporte un miroir semi-transparent, pour brancher une partie du signal optique pour la mesure vers le capteur de mesure.

**10.** Appareil testeur selon l'une des revendications 1 à 9, **caractérisé en ce qu'**il comprend en outre un câble de guidage de lumière, qui est disposé en amont de la LED UV à rediriger le signal optique émis, de sorte que le signal optique, de préférence dans le but d'une simulation masquée, peut être amené à proximité du capteur du système d'alerte.

A)

1    2    3

4

Optisches Ausgangsspektrum

▷ → ⊗ ⋯▶ Shutter oder Sensor ⋯▶

Lampe

Regelung oder Steuerung

Power

5

UV-C     Wavelenght

B)

1

6

7

Optisches Ausgangsspektrum

▷ → ⟠ ⋯▶

LED

Power

8

UV-C     Wavelenght

## Fig. 1

IR / UV
Intensität

Missile Profil (Beispiel)

Zeit

## Fig. 2

Intensität

30

32

UV-Quelle ⊗

MAWS Tester (MAWST)

31

Anzeige im Cockpit

35

○ ○ ○ ○ ○

33

MAWS

Missile Approach
Warning Sensor

EWC
(Electronic Warfare
Computer)

34

## Fig. 3

42

43

40

45

Taste

Verstärker

41

46

UV- LED

Batterie

Microcontroller

Strom und Spannung
werden zurückgeführt bzw.
überwacht

44

47

Shunt

## Fig. 4

50

51

Microcontroller

Sollwert (S)
aus EEPROM → ( ) Regelabweichung
(D=S-I) → | Regler | Stellgrösse
(U=f(D)) → Regelstrecke
(UV-LED)

−

Istwerte (I)

Fig. 5

### Intensitätsverlauf 240 bis 290nm

Fig. 6

70

72

**Fig. 7**

73

71

82

Taste

Ext.
10Bit
D/A-
Wandler

Verstärker

81

Optik

UV- LED

Batterie

Microcontroller

I2C-Bus

Ext.
10Bit
A/D-
Wandler

Rückführung von
Strom, Spannung
und Lichtleistung

Shunt

UVC-
Sensor

83

80

**Fig. 8**

$$P_S \approx l^2 \cdot P_E$$

## Fig. 9

## Fig. 10

# Main

Initialisierung

BIT (Built- in- test) ausführen

BIT Error

ja                                                                nein

Durch Taste eingeschaltet
(Normal- Modus)

ja                                            nein

Selbsthaltung aktivieren

10V Speisung einschalten

BIT ausführen

BIT OK

Profil generieren
ev. Regelung
(alle 10ms neuer Wert)

BIT ausführen

Zustand an Bicolor LED
und Vibrator darstellen

Break wenn Profil beendet

Alle Werte auf zurücksetzen

Zustand Profil beendet
an Bicolor LED
darstellen

Selbsthaltung deaktivieren

Fehler anzeigen

Kalibration

# Fig. 11

14

## Initialisierung

| |
|---|
| Controller Einstellungen und include von Header-Files |
| Interner Oszillator aktivieren (ev. PLL) |
| Ports definieren |
| Timer einstellen |
| ADC definieren |
| PWM definieren |
| Einstellungen RS232 |
| RTI (Real Time Interrupt) einstellen |
| Interrupts aktivieren |

Fig. 12

## BIT

| |
|---|
| Batteriespannung überprüfen |
| ev. /LBO einlesen (Low Battery vom Step-Up Conv.) |
| Temperatur messen |
| UV- LED Strom messen |
| UV- LED Spannung messen |

Fig. 13

**IN DER BESCHREIBUNG AUFGEFÜHRTE DOKUMENTE**

**In der Beschreibung aufgeführte Patentdokumente**

- US 20050150371 A1
- US 20050029394 A1
- US 20040174290 A1
- US 5850285 A
- US 5693951 A